# EUROPEAN PATENT APPLICATION

(11) **EP 4 563 577 A1**
(43) Date of publication of application: **04.06.2025**
(21) Application number: 23847049.6
(22) Date of filing: 28.07.2023
(51) Int. Cl.: C07D 471/04, A61K 31/437, A61P 1/00

(54) **NOVEL SALT OF IMIDAZO[1,2-A]PYRIDINE COMPOUND, CRYSTALLINE FORM THEREOF, AND PREPARATION METHOD**

(30) Priority: 29.07.2022 KR 20220095088
(71) Applicant: Jeil Pharmaceutical Co., Ltd., Seoul 06543 (KR); Onconic Therapeutics Inc., Gangnam-gu Seoul 06236 (KR)
(72) Inventor: JEON, Seong Hyeon, Yongin-si Gyeonggi-do 17172 (KR); AN, Jung Gi, Yongin-si Gyeonggi-do 17172 (KR); HONG, Seung Min, Yongin-si Gyeonggi-do 17172 (KR); KIM, Sung Hwa, Yongin-si Gyeonggi-do 17172 (KR)
(74) Representative: HGF
(86) International application number: PCT/KR2023/011079
(87) International publication number: WO 2024/025393

(57) **Abstract**

In a novel salt of imidazo[1,2-a]pyridine compound of the present invention, a Crystalline Form thereof, and a method for preparing the same, the novel salt of imidazo[1,2-a]pyridine compound is azetidin-1-yl{8-[(2,6-dimethylbenzyl)amino]-2,3-dimethylimidazo[1,2-a]pyridin-6-yl}methanone citrate.

## Description

### Technical Field

The present invention relates to a novel salt of imidazo[1,2-a]pyridine compound, a crystalline form thereof, and preparation method.

### Background

Gastrointestinal inflammatory diseases or gastric acid-related diseases such as peptic ulcer, gastric/duodenal ulcer, gastritis, gastroesophageal reflux disease (GERD), non-erosive reflux disease (NERD), etc., are the most common digestive troubles afflicted by most of the world's population, including Korea.

In order to solve the problem of conventional proton pump inhibitors (PPIs), there are recently growing interest in and need for potassium competitive acid blocker (P-CAB, acid pump antagonist) drugs having a mechanism of reversibly binding to K⁺ binding site of H⁺/K⁺-ATPase to inhibit acid secretion through potassium-competitive inhibition among proton pump inhibitors (PPIs). In particular, unlike an irreversible proton pump inhibitor (PPI), it is expected that a reversible proton pump inhibitor (P-CAB) has rapid efficacy in terms of mechanism, is easily taken no matter when it is administered before and after meals, and is very effective in ameliorating symptoms at night, which are a problem of the irreversible proton pump inhibitor.

Meanwhile, a compound needs to have not only preferable biological properties, but also physical properties enough to allow a use thereof in preparing a pharmaceutical composition such that the compound may be considered as a candidate material for being developed as a drug. In addition, it is preferable that this compound has a solid phase in order to be easily prepared and stably formulated.

Accordingly, the present inventors have made intensive research efforts to find a form which has stability in various aspects and may be industrialized in order to use the imidazo[1,2-a]pyridine compound in a pharmaceutical manner, and as a result, have confirmed a novel salt exhibiting a remarkable effect that could not be predicted, thereby completing the present invention.

### Related Art References

### Patent Documents

Korean Registered Patent Publication No. 10-1777971

### Detailed Description of the Invention

### Technical Problem

One object of the present invention is to provide a novel salt of imidazo[1,2-a]pyridine compound.

Another object of the present invention is to provide a crystalline form of a novel salt of imidazo[1,2-a]pyridine compound.

Still another object of the present invention is to provide a method for preparing a novel salt of imidazo[1,2-a]pyridine compound.

### Technical Solution

### Novel salt of imidazo[1,2-alpyridine compound

(1) A novel salt of imidazo[1,2-a]pyridine compound according to the present invention is azetidin-1-yl{8-[(2,6-dimethylbenzyl)amino]-2,3-dimethylimidazo[1,2-a]pyridin-6-yl }methanone citrate.

Hereinafter, the term "citrate of chemical formula I" or "citrate" simply used herein all means "azetidin-1-yl{8-[(2,6-dimethylbenzyl)amino]-2,3-dimethylimidazo[1,2-a]pyridin-6-yl}methanone citrate."

Azetidin-1-yl{8-[(2,6-dimethylbenzyl)amino]-2,3-dimethylimidazo[1,2-a]pyridin-6-yl}methanone is represented by chemical formula I below:

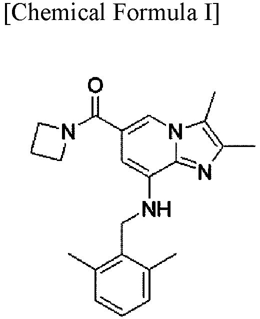

**(2)** In above **(1),** in azetidin-1-yl{8-[(2,6-dimethylbenzyl)amino]-2,3-dimethylimidazo[1,2-a]pyridin-6-yl}methanone citrate of the citrate, a molar ratio of azetidin-1-yl{8-[(2,6-dimethylbenzyl)amino]-2,3-dimethylimidazo[1,2-a]pyridin-6-yl}methanone and citric acid may be 1:0.3 to 1:1.3. As one example, the molar ratio may be 1:0.5 to 1:1.

**(3)** In above **(1)** or **(2),** azetidin-1-yl{8-[(2,6-dimethylbenzyl)amino]-2,3-dimethylimidazo[1,2-a]pyridin-6-yl}methanone citrate of the present invention may be represented by chemical formula II below:

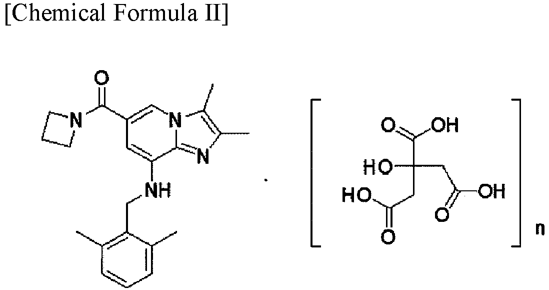

In above chemical formula II, n represents 0.3 to 1.3.

In one embodiment, n of above chemical formula II may be 0.5 to 1.

**(4)** In any one of above **(1)** to **(3),** azetidin-1-yl{8-[(2,6-dimethylbenzyl)amino]-2,3-dimethylimidazo[1,2-a]pyridin-6-yl}methanone citrate may be anhydrous.

**(5)** In any one of above **(1)** to **(4),** azetidin-1-yl{8-[(2,6-dimethylbenzyl)amino]-2,3-dimethylimidazo[1,2-a]pyridin-6-yl}methanone citrate may be crystalline or amorphous.

**(6)** In any one of above **(1)** to **(5),** azetidin-1-yl{8-[(2,6-dimethylbenzyl)amino]-2,3-dimethylimidazo[1,2-a]pyridin-6-yl}methanone citrate may be anhydrous crystalline.

**(7)** In any one of above **(1)** to **(6),** azetidin-1-yl{8-[(2,6-dimethylbenzyl)amino]-2,3-dimethylimidazo[1,2-a]pyridin-6-yl}methanone citrate may be Crystalline Form A which shows a powder X-ray diffraction pattern including at least three diffraction peaks where a 2θ (±0.2°) value of the powder X-ray diffraction pattern is selected from the group consisting of 5.46°, 7.14°, 10.61°, 11.82°, 18.27° and 25.77°.

**(8)** In above **(7),** the powder X-ray diffraction pattern of Crystalline Form A may further include at least one diffraction peak where the 2θ (±0.2°) value is selected from the group consisting of 10.93°, 13.11°, 14.15°, 15.84°, 16.35°, 19.79° and 24.21°.

**(9)** In above **(7)** or **(8),** Crystalline Form A may have a differential scanning calorific (DSC) endothermic peak at 88.69°C, 135.61°C and 154.84°C (±0.5°C) when a heating rate is 10°C/min.

**(10)** In any one of above **(1)** to **(6),** azetidin-1-yl{8-[(2,6-dimethylbenzyl)amino]-2,3-dimethylimidazo[1,2-a]pyridin-6-yl}methanone citrate may be Crystalline Form B which shows a powder X-ray diffraction pattern including at least three diffraction peaks where a 2θ (±0.2°) value of the powder X-ray diffraction pattern is selected from the group consisting of 7.03°, 7.69°, 9.47°, 13.21°, 14.86° and 21.13°.

**(11)** In above **(10),** Crystalline Form B may have a differential scanning calorific (DSC) endothermic peak at 144.57°C (±0.5°C) when a heating rate is 10°C/min.

**(12)** In any one of above **(1)** to **(6),** azetidin-1-yl{8-[(2,6-dimethylbenzyl)amino]-2,3-dimethylimidazo[1,2-a]pyridin-6-yl}methanone citrate may be Crystalline Form C1 which shows a powder X-ray diffraction pattern including at least three diffraction peaks where a 2θ (±0.2°) value of the powder X-ray diffraction pattern is selected from the group consisting of 7.35°, 15.31°, 17.42° and 22.26°.

**(13)** In above **(12),** the powder X-ray diffraction pattern of Crystalline Form C1 may further include at least one diffraction peak where the 2θ (±0.2°) value is selected from the group consisting of 10.23°, 12.90°, 14.68°, 15.97°, 18.21°, 21.22° and 26.00°.

**(14)** In above **(12)** or **(13),** Crystalline Form C1 may have a differential scanning calorific (DSC) endothermic peak at 168.93°C (±0.5°C) when a heating rate is 10°C/min.

**(15)** In any one of above **(1)** to **(6),** azetidin-1-yl{8-[(2,6-dimethylbenzyl)amino]-2,3-dimethylimidazo[1,2-a]pyridin-6-yl}methanone citrate may be Crystalline Form C2 which shows a powder X-ray diffraction pattern including at least three diffraction peaks where a 2θ (±0.2°) value of the powder X-ray diffraction pattern is selected from the group consisting of 5.63°, 8.90°, 9.51° and 13.01°.

**(16)** In above **(15),** the powder X-ray diffraction pattern of Crystalline Form C2 may further include at least one diffraction peak where the 2θ (±0.2°) value is selected from the group consisting of 12.31°, 14.34°, 14.80°, 18.38°, 18.75° and 19.62°.

**(17)** In above **(15)** or **(16),** Crystalline Form C2 may have a differential scanning calorific (DSC) endothermic peak at 161.48°C (±0.5°C) when a heating rate is 10°C/min.

**(18)** In any one of above **(1)** to **(5),** amorphous form of azetidin-1-yl{8-[(2,6-dimethylbenzyl)amino]-2,3-dimethylimidazo[1,2-a]pyridin-6-yl}methanone citrate may have a differential scanning calorific (DSC) endothermic peak at 161.42°C (±0.5°C) when a heating rate is 10°C/min.

**(19)** In any one of above **(1)** to **(11),** in each of Crystalline Forms A and B of citrate according to the present invention, a molar ratio of azetidin-1-yl{8-[(2,6-dimethylbenzyl)amino]-2,3-dimethylimidazo[1,2-a]pyridin-6-yl}methanone and citric acid may be 1:0.5.

**(20)** In any one of above **(1)** to **(6)** and **(12)** to **(17),** in each of Crystalline Forms C1 and C2 of citrate according to the present invention, a molar ratio of azetidin-1-yl{8-[(2,6-dimethylbenzyl)amino]-2,3-dimethylimidazo[1,2-a]pyridin-6-yl}methanone and citric acid may be 1:1.

Azetidin-1-yl{8-[(2,6-dimethylbenzyl)amino]-2,3-dimethylimidazo[1,2-a]pyridin-6-yl}methanone citrate according to the present invention may exhibit an unexpected very excellent bioavailability, and thus may be used as an active ingredient of the pharmaceutical composition, so as to exhibit a very excellent effect in preventing or treating gastrointestinal inflammatory diseases or gastric acid-related diseases.

In addition, azetidin-1-yl{8-[(2,6-dimethylbenzyl)amino]-2,3-dimethylimidazo[1,2-a]pyridin-6-yl}methanone citrate according to the present invention may have advantages of being easily prepared through a simple process and obtained with high purity at a high yield of 90% or more.

Furthermore, azetidin-1-yl{8-[(2,6-dimethylbenzyl)amino]-2,3-dimethylimidazo[1,2-a]pyridin-6-yl }methanone citrate according to the present invention may be used to formulate drug products since various Crystalline Forms are generated due to minute differences in reaction temperature, reaction rate, etc., even under similar solvent conditions and these Crystalline Forms have excellent photostability, heat/moisture stability, long-term storage stability, etc.

A pharmaceutical composition of the present invention includes a therapeutically effective amount of azetidin-1-yl{8-[(2,6-dimethylbenzyl)amino]-2,3-dimethylimidazo[1,2-a]pyridin-6-yl}methanone citrate.

The pharmaceutical composition of the present invention may treat or prevent diseases selected from the group consisting of peptic ulcer, gastric/duodenal ulcer, non-steroidal antiinflammatory drug (NSAID)-induced ulcer, helicobacter pylori infection, functional dyspepsia, Zollinger-Ellison syndrome, gastritis, gastroesophageal reflux disease (GERD), and non-erosive reflux disease (NERD).

### Method for preparing novel salt of imidazo[1,2-a]pyridine compound

**(21)** A method for preparing azetidin-1-yl{8-[(2,6-dimethylbenzyl)amino]-2,3-dimethylimidazo[1,2-a]pyridin-6-yl}methanone citrate according to the present invention includes preparing citrate by reacting azetidin-1-yl{8-[(2,6-dimethylbenzyl)amino]-2,3-dimethylimidazo[1,2-a]pyridin-6-yl}methanone and citric acid.

**(22)** In the preparing of citrate according to above **(21),** azetidin-1-yl{8-[(2,6-dimethylbenzyl)amino]-2,3-dimethylimidazo[1,2-a]pyridin-6-yl}methanone and citric acid may be reacted in a presence of at least one solvent selected from an alcohol having one to three carbon atoms, acetone, acetonitrile, tetrahydrofuran (THF), dichloromethane, dimethylformamide (DMF), N-methylpyrrolidone (NMP), and purified water.

**(23)** In above **(21)** or **(22),** azetidin-1-yl{8-[(2,6-dimethylbenzyl)amino]-2,3-dimethylimidazo[1,2-a]pyridin-6-yl}methanone may be synthesized according to reaction formula 1 below:

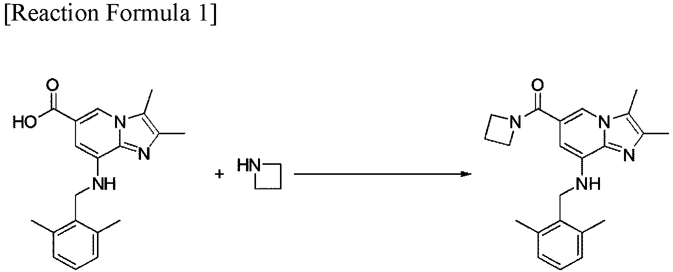

**(24)** In above reaction formula 1 according to above **(23),** 8-(2,6-dimethylbenzylamino)-2,3-dimethylimidazo[1,2-a]pyridin-6-carboxylic acid may be obtained as described in Korean Registered Patent Publication No. 10-1777971, and a specific process of above reaction formula 1 may also be performed according to the contents disclosed therein.

**(25)** In any one of above **(21)** to **(24),** in the preparing of citrate, azetidin-1-yl{8-[(2,6-dimethylbenzyl)amino]-2,3-dimethylimidazo[1,2-a]pyridin-6-yl}methanone may be a non-solvate, a solvate, or a mixture thereof.

**(26)** In any one of above **(23)** to **(25),** the solvate, the non-solvate, or the mixture thereof obtained by crystallizing the azetidin-1-yl{8-[(2,6-dimethylbenzyl)amino]-2,3-dimethylimidazo[1,2-a]pyridin-6-yl}methanone obtained according to above reaction formula 1 in an alcohol solvent having one to three carbon atoms, followed by vacuum-drying may be reacted with citric acid. The solvate of azetidin-1-yl{8-[(2,6-dimethylbenzyl)amino]-2,3-dimethylimidazo[1,2-a]pyridin-6-yl}methanone may be obtained by vacuum-drying at about 20°C or higher to about 35°C or lower. The non-solvate of azetidin-1-yl{8-[(2,6-dimethylbenzyl)amino]-2,3-dimethylimidazo[1,2-a]pyridin-6-yl}methanone may be obtained by vacuum-drying at about 45°C or higher and about 60°C or lower. The mixture of the solvate and non-solvate may be obtained by vacuum-drying at a temperature of more than about 35°C and less than about 45°C.

**(27)** In any one of above **(21)** to **(26),** in the preparing of citrate, citrate where azetidin-1-yl{8-[(2,6-dimethylbenzyl)amino]-2,3-dimethylimidazo[1,2-a]pyridin-6-yl}methanone and citric acid are included at a molar ratio of about 1:0.3 to about 1:1.3 may be prepared. For example, in the preparing of citrate, citrate where a molar ratio of azetidin-1-yl{8-[(2,6-dimethylbenzyl)amino]-2,3-dimethylimidazo[1,2-a]pyridin-6-yl}methanone and citric acid is about 1:1 or about 1:0.5 may be prepared.

**(28)** In any one of above **(21)** to **(27),** in the preparing of citrate, the solvate, the non-solvate, or the mixture thereof obtained from azetidin-1-yl{8-[(2,6-dimethylbenzyl)amino]-2,3-dimethylimidazo[1,2-a]pyridin-6-yl}methanone and citric acid may be mixed to have a weight ratio of about 1:1.3 to about 1:0.3.

**(29)** In any one of above **(21)** to **(28),** the preparing of citrate may include mixing a first solution including the solvate, the non-solvate, or the mixture thereof obtained from azetidin-1-yl{8-[(2,6-dimethylbenzyl)amino]-2,3-dimethylimidazo[1,2-a]pyridin-6-yl}methanone and a first solvent, and a second solution including citric acid and a second solvent.

**(30)** In above **(29),** the first solution may be first stirred in a reactor, and then the second solution may be added, or the second solution may be first stirred in the reactor, and then the first solution may be added.

**(31)** In above **(29)** or **(30),** the first and second solvents may each independently include at least one solvent selected from an alcohol having one to three carbon atoms, acetone, acetonitrile, THF, dichloromethane, DMF, NMP, and purified water.

**(32)** In any one of above **(29)** to **(31),** the first solvent may be a single solvent of purified water or a mixed solvent including at least one organic solvent selected from an alcohol having one to three carbon atoms, acetone, acetonitrile, THF, dichloromethane, DMF, and NMP together with purified water. In this case, the second solvent may be a single solvent of purified water or a mixed solvent including at least one organic solvent selected from an alcohol having one to three carbon atoms, acetone, acetonitrile, THF, dichloromethane, DMF, and NMP together with purified water. Under the condition of the first and second solvents, citrate according to the present invention may be Crystalline Form A.

**(33)** In any one of above **(29)** to **(31),** the first solvent may be an organic solvent, and the second solvent may be a single solvent of purified water, or a mixed solvent including at least one organic solvent together with purified water. Under the condition of the first and second solvents, citrate according to the present invention may be Crystalline Form A.

**(34)** In any one of above **(29)** to **(31),** the first solvent may be an organic solvent, the second solvent may be an organic solvent, and the first and second solvents may be the same as each other. Citrate according to the present invention may be Crystalline Form B under the condition that the first and second solvents are alcohols having one to three carbon atoms as an organic solvent.

**(35)** In any one of above **(29)** to **(31),** the first solvent may be acetone or a mixed solvent in which at least one organic solvent other than acetone and acetone are mixed, and the second solvent may be acetone. Under the condition of the first and second solvents, citrate according to the present invention may be Crystalline Form C1 or Crystalline Form C2.

**(36)** In any one of above **(21)** to **(33),** in the preparing of citrate, azetidin-1-yl{8-[(2,6-dimethylbenzyl)amino]-2,3-dimethylimidazo[1,2-a]pyridin-6-yl}methanone and citric acid may be reacted at about 10°C to about 30°C, preferably at about 20°C to about 25°C, and a subsequent stirring process may be continuously performed at the same temperature. Under the condition of the temperatures, citrate according to the present invention may be Crystalline Form A.

**(37)** In any one of above **(21)** to **(31)** and **(34),** in the preparing of citrate, azetidin-1-yl{8-[(2,6-dimethylbenzyl)amino]-2,3-dimethylimidazo[1,2-a]pyridin-6-yl}methanone and citric acid may be reacted at about 10°C to about 30°C, preferably at about 20°C to about 25°C, and then cooled down and stirred at about 0°C to about 10°C, preferably at about 0°C to about 5°C. Under the condition of the process, citrate according to the present invention may be Crystalline Form B.

**(38)** In any one of above **(21)** to **(31)** and **(35),** in the preparing of citrate, azetidin-1-yl{8-[(2,6-dimethylbenzyl)amino]-2,3-dimethylimidazo[1,2-a]pyridin-6-yl}methanone and citric acid may be reacted at about 45°C to about 60°C, preferably at about 50°C to about 55°C, and then cooled down and stirred at about 10°C to about 30°C, preferably at about 20°C to about 25°C. Under the condition of the process, citrate according to the present invention may be Crystalline Form C1.

**(39)** In any one of above **(21)** to **(31)** and **(35),** in the preparing of citrate, azetidin-1-yl{8-[(2,6-dimethylbenzyl)amino]-2,3-dimethylimidazo[1,2-a]pyridin-6-yl}methanone and citric acid may be reacted at about 10°C to about 30°C, preferably at about 20°C to about 25°C, and then cooled down and stirred at about 0°C to about 10°C, preferably at about 0°C to about 5°C. Here, it may be performed at about 10°C to about 30°C within a short time in which time required for introducing citric acid into azetidin-1-yl{8-[(2,6-dimethylbenzyl)amino]-2,3-dimethylimidazo[1,2-a]pyridin-6-yl}methanone is set to 15 minutes, preferably 10 minutes. Under the condition of the process, the citrate may be obtained as Crystalline Form C1.

**(40)** In any one of above **(21)** to **(31)** and **(35),** in the preparing of citrate, azetidin-1-yl{8-[(2,6-dimethylbenzyl)amino]-2,3-dimethylimidazo[1,2-a]pyridin-6-yl}methanone and citric acid may be reacted at about 10°C to about 30°C, preferably at about 20°C to about 25°C, and then heated and stirred at about 45°C to about 60°C, preferably at about 50°C to about 55°C, and then cooled down and stirred at about 10°C to about 30°C, preferably at about 20°C to about 25°C. Here, time required for introducing citric acid into azetidin-1-yl{8-[(2,6-dimethylbenzyl)amino]-2,3-dimethylimidazo[1,2-a]pyridin-6-yl}methanone may be at least 30 minutes, preferably at least 60 minutes. Under the condition of the process, citrate according to the present invention may be Crystalline Form C2.

**(41)** In any one of above **(21)** to **(40),** the preparing of citrate may include: filtering a solid, that is, citrate, produced from a reaction between azetidin-1-yl{8-[(2,6-dimethylbenzyl)amino]-2,3-dimethylimidazo[1,2-a]pyridin-6-yl}methanone and citric acid; washing; and drying. The filtering and the washing may each be performed by a method performed in a conventional salt preparation. The drying may be performed at about 35°C to about 50°C, preferably about 35°C to about 45°C, and may be performed in a vacuum-drying or nitrogen-drying manner.

### Advantageous Effects

In a novel salt of imidazo[1,2-a]pyridine compound of the present invention, a Crystalline Form thereof, and a method for preparing the same, citrate of imidazo[1,2-a]pyridine compound of the present invention may exhibit an unexpected very excellent bioavailability, and thus may be used as an active ingredient of the pharmaceutical composition, so as to exhibit a very excellent effect in preventing or treating gastrointestinal inflammatory diseases or gastric acid-related diseases.

In addition, azetidin-1-yl{8-[(2,6-dimethylbenzyl)amino]-2,3-dimethylimidazo[1,2-a]pyridin-6-yl}methanone citrate according to the present invention may have advantages of being easily prepared through a simple process and obtained with high purity at a high yield of 90% or more.

Furthermore, azetidin-1-yl{8-[(2,6-dimethylbenzyl)amino]-2,3-dimethylimidazo[1,2-a]pyridin-6-yl }methanone citrate according to the present invention may be used to formulate drug products since various Crystalline Forms are generated due to minute differences such as reaction temperature, reaction rate, etc., even under similar solvent conditions and these Crystalline Forms have excellent photostability, heat/moisture stability, long-term storage stability, etc.

### Brief Description of the Drawings

FIGS. 1, 2, and 3 are views showing results of XRD, DSC, and TGA analysis on Crystalline Form A of citrate according to the present invention, respectively.
FIGS. 4, 5, and 6 are views showing results of XRD, DSC, and TGA analysis on Crystalline Form B of citrate according to the present invention, respectively.
FIGS. 7, 8, and 9 are views showing results of XRD, DSC, and TGA analysis on Crystalline Form C1 of citrate according to the present invention, respectively.
FIGS. 10, 11, and 12 are views showing results of XRD, DSC, and TGA analysis on Crystalline Form C2 of citrate according to the present invention, respectively.
FIGS. 13, 14, and 15 are views showing results of XRD, DSC, and TGA analysis on amorphous form of citrate according to the present invention, respectively.
FIG. 16 is a view showing results of XRD analysis on citrate according to the present invention after 36 months of storage.

### Mode for Invention

Hereinafter, all the terms used herein including technical or scientific terms have the same meaning as commonly understood by those ordinary skilled in the art, to which the present invention pertains, unless defined otherwise. Such terms as those defined in a generally used dictionary are to be interpreted to have the meanings equal to the contextual meanings in the relevant art, and are not to be interpreted to have ideal or excessively formal meanings, unless clearly defined in the present application.

### <Measurement Method>

A following measurement method may be commonly applied to each of Examples according to the present invention.

### 1. X-ray powder diffraction (XPRD)

An X-ray powder diffraction pattern was obtained through a solid phase detector over a range of 2° to 40° of a diffraction angle (2θ) with a 0.02° step size by using D8 Focus (Bruker ASX).

### 2. Thermal analysis (DSC)

A differential scanning calorimetry (DSC) was performed by using DSC 8000 (PerkinElmer). Samples were evaluated by using a linear heating ramp of 10°C/min in the range of 30°C to 300°C.

### 3. Thermogravimetric analysis (TGA)

A thermogravimetric analysis was performed at 30°C to 900°C by using TGA 8000 (PerkinElmer), in which 0.5 mg to 2 mg of samples were weighed into a ceramic crucible and measured under the condition of 5°C/min.

### 4. NMR analysis

A nuclear magnetic resonance (NMR) analysis was performed by using Varian Mercury 400.

### 5. Moisture content measurement/analysis

The moisture content of the obtained sample was measured with an 870KF Titrino Plus (Metrohm) Karl-Fischer moisture meter.

### Preparation Example 1: Synthesis of compound I

Azetidin-1-yl{8-[(2,6-dimethylbenzyl)amino]-2,3-dimethylimidazo[1,2-a]pyridin-6-yl}methanone was synthesized in the same manner as in the preparation method of the compound of Example 6 in Korean Registered Patent Publication No. 10-1777971.

¹H NMR (400 MHz, CDCl₃); *δ*7.63(d, *J*=1.2 Hz, 1H), 7.13(dd, *J* =8.4, 6.8 Hz, 1H), 7.06-7.04(m, 2H), 6.42(d, *J=* 1.2 Hz, 1H), 4.86-4.84(m, 1H), 4.41-4.28(m, 4H), 4.37(d, J=4.4 Hz, 2H), 3.75-3.69(m, 1H), 2.43-2.34(m, 13H)

### Preparation Example 2: Preparation of solvate of compound I

Azetidin-1-yl{8-[(2,6-dimethylbenzyl)amino]-2,3-dimethylimidazo[1,2-a]pyridin-6-yl}methanone obtained in Preparation Example 1 was crystallized in an alcohol solvent (isopropyl alcohol, IPA) and dried under vacuum at about 30°C to 35°C, so as to obtain a solvate of azetidin-1-yl{8-[(2,6-dimethylbenzyl)amino]-2,3-dimethylimidazo[1,2-a]pyridin-6-yl}methanone.

### Example 1(1): Preparation of compound I citrate - Crystalline Form A

To a reactor, 10 g of the solvate of compound I obtained in above Preparation Example 2 and 211 g of purified water or a mixed solvent (dichloromethane, DMF, NMP, or acetone) with purified water were added, and then stirred at 20°C to 25°C for 10 minutes. Subsequently, a solution prepared by dissolving 42.3 g of purified water in 4.9 g of citric acid was added at about 20°C to 25°C to confirm that a solid was produced, and then continuously stirred at the same temperature for two hours, after which the resulting solid was filtered, washed with 25.4 g of purified water, and dried under vacuum or dried under nitrogen at about 40°C, so as to prepare 9.70 g of azetidin-1-yl{8-[(2,6-dimethylbenzyl)amino]-2,3-dimethylimidazo[1,2-a]pyridin-6-yl}methanone citrate.

### Example 1(2): Preparation of compound I citrate - Crystalline Form A

A solution prepared by dissolving 16.9 g of purified water or a mixed solvent of purified water (acetone, DMF, or NMP) in 9.8 g of citric acid was added to a reactor, and then stirred at 20°C to 25°C for 10 minutes. Subsequently, a solution prepared by dissolving 90 g of dichloromethane in 20 g of the solvate of compound I obtained in above Preparation Example 2 was added at the same temperature to confirm that a solid was produced, and then continuously stirred at the same temperature for two hours, after which the resulting solid was filtered, washed with 50.0 g of purified water, and dried under vacuum or dried under nitrogen at about 40°C, so as to prepare 19.4 g of azetidin-1-yl{8-[(2,6-dimethylbenzyl)amino]-2,3-dimethylimidazo[1,2-a]pyridin-6-yl}methanone citrate.

### Analysis 1 - Results of XRD, DSC, and TGA measurement of Crystalline Form A

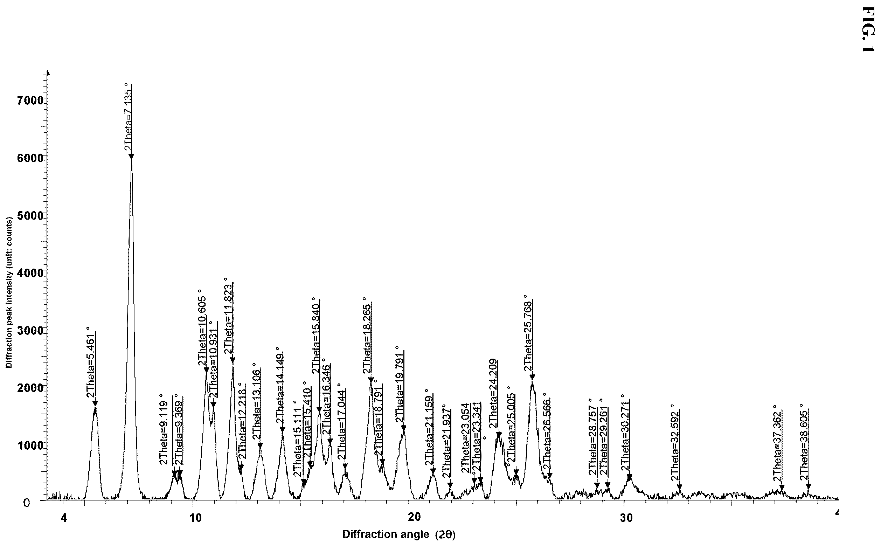

Referring to FIG. 1 and table 1 below, a 2θ value (unit: °) of the XRD pattern of Crystalline Form A according to the present invention can be confirmed. Specifically, the XRD pattern of Crystalline Form A according to the present invention includes a diffraction peak where a 2θ value is 5.46°, 7.14°, 10.61°, 10.93°, 11.82°, 13.11°, 14.15°, 15.84°, 16.35°, 18.27°, 19.79°, 24.21° and 25.77°.

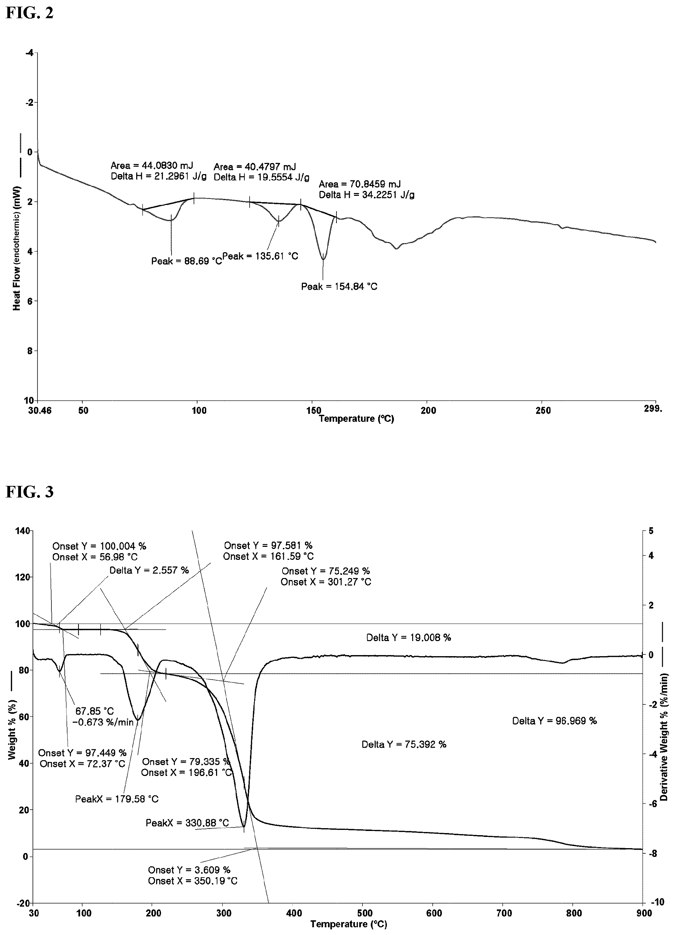

Referring to FIG. 2, Crystalline Form A shows a DSC endothermic peak at 88.69°C, 135.61°C, and 154.84°C.

Referring to FIG. 3, as a result of measurement by TGA, it is confirmed that Crystalline Form A is decomposed in three steps. Specifically, it was found that decomposition occurs in a first step of about 56.98°C to 72.37°C, a second step of about 161.59°C to 196.61°C, and a third step of about 301.27°C to 350.19°C, and a maximum inflection point to be decomposed is confirmed at about 67.85°C, 179.58°C, and 330.88°C, respectively.

Meanwhile, it can be confirmed through a moisture content that Crystalline Form A is anhydrous.

In addition, it was confirmed that Crystalline Form A according to the present invention is one in which azetidin-1-yl{8-[(2,6-dimethylbenzyl)amino]-2,3-dimethylimidazo[1,2-a]pyridin-6-yl}methanone and citric acid are bonded at a molar ratio of about 1:0.5.

**[Table 1]**

| **Diffraction angle (2θ, unit: °)** | **Relative intensity** |
|---|---|
| 5.46 | Strong |
| 7.14 | Strong |
| 9.12 | Weak |
| 9.37 | Weak |
| 10.61 | Strong |
| 10.93 | Moderate |
| 11.82 | Strong |
| 13.11 | Moderate |
| 14.15 | Moderate |
| 15.84 | Moderate |
| 16.35 | Moderate |
| 17.04 | Weak |
| 18.27 | Strong |
| 19.79 | Moderate |
| 21.16 | Weak |
| 24.21 | Moderate |
| 25.77 | Strong |

### Example 2: Preparation of compound I citrate - Crystalline Form B

To a reactor, 166.2 g of IPA and 10 g of the solvate of compound I obtained in above Preparation Example 2 were added, and then stirred for 10 minutes. Subsequently, a solution in which 33.2 g of IPA was dissolved in 4.9 g of citric acid was added at 20°C to 25°C so as to confirm that a solid was produced, and then continuously stirred at the same temperature for one hour, cooled down to 0°C to 5°C and further stirred for one hour. The resulting solid was filtered and washed with 19.9 g of IPA so as to obtain 9.63 g of azetidin-1-yl{8-[(2,6-dimethylbenzyl)amino]-2,3-dimethylimidazo[1,2-a]pyridin-6-yl}methanone citrate.

### Analysis 2 - Results of XRD, DSC, and TGA measurement of Crystalline Form B

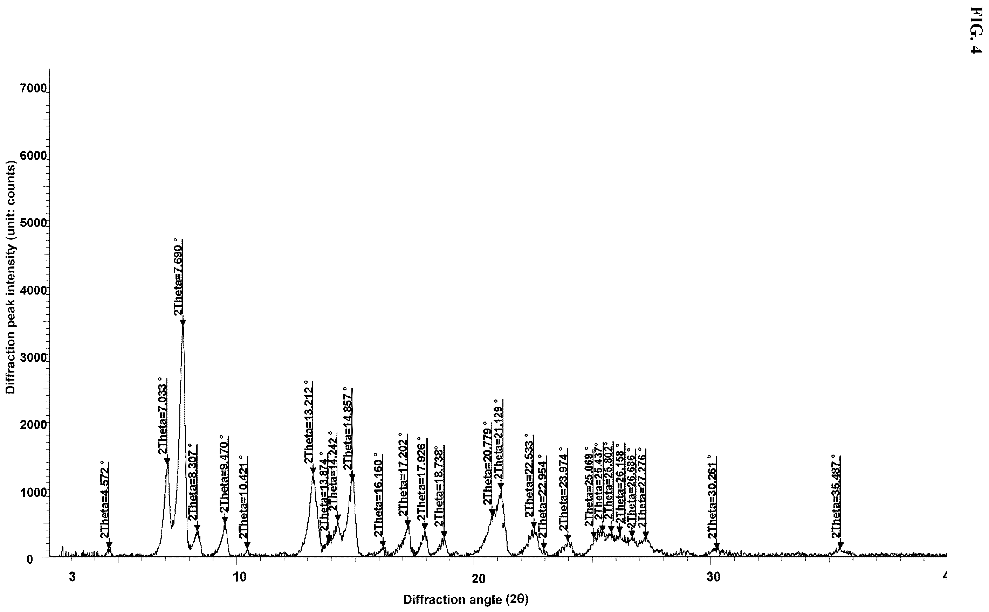

Referring to FIG. 4 and table 2 below, a 2θ value (unit: °) of the XRD pattern of Crystalline Form B according to the present invention may be confirmed. Specifically, the XRD pattern of Crystalline Form B according to the present invention includes a diffraction peak where a 2θ value is 7.03°, 7.69°, 9.47°, 13.21°, 14.86° and 21.13°.

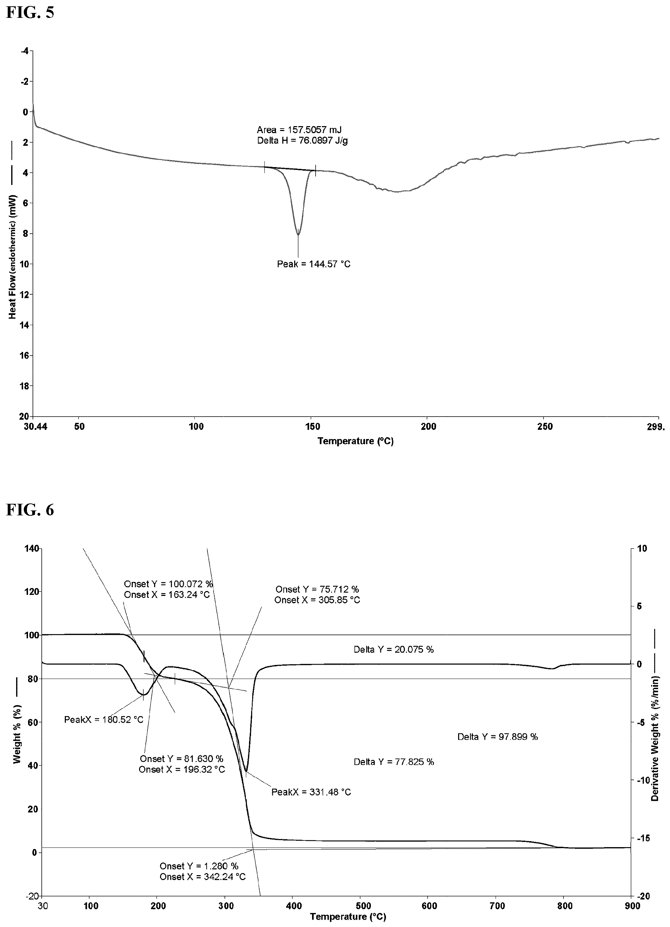

Referring to FIG. 5, Crystalline Form B shows a DSC endothermic peak at 144.57°C.

Referring to FIG. 6, as a result of TGA analysis, it is confirmed that Crystalline Form B is decomposed in two steps. Specifically, it was found that decomposition occurs in a first step of about 163.24°C to 196.32°C and a second step of about 305.85°C to 342.24°C, and a maximum inflection point to be decomposed is confirmed at about 180.52°C and 331.48°C, respectively.

Meanwhile, it can be confirmed through a moisture content that Crystalline Form B is anhydrous.

In addition, it was confirmed that Crystalline Form B according to the present invention is one in which azetidin-1-yl{8-[(2,6-dimethylbenzyl)amino]-2,3-dimethylimidazo[1,2-a]pyridin-6-yl}methanone and citric acid are bonded at a molar ratio of about 1:0.5.

**[Table 2]**

| **Diffraction angle (2θ, unit: °)** | **Relative intensity** |
|---|---|
| 7.03 | Strong |
| 7.69 | Strong |
| 8.31 | Weak |
| 9.47 | Moderate |
| 13.21 | Strong |
| 14.24 | Weak |
| 14.86 | Strong |
| 17.20 | Weak |
| 17.93 | Weak |
| 18.74 | Weak |
| 21.13 | Strong |
| 22.53 | Weak |
| 23.97 | Weak |

### Example 3(1): Preparation of compound I citrate - Crystalline Form C1

To a reactor, 167.2 g of acetone and 10 g of the solvate of compound I obtained in above Preparation Example 2 were added, and then heated up to 50°C to 55°C and stirred for 10 minutes. Subsequently, a solution in which 33.4 g of acetone was dissolved in 4.9 g of citric acid was added and stirred at the same temperature for one hour, and then cooled down to 20°C to 25°C and further stirred for one hour. The resulting solid was filtered and washed with 20.1 g of acetone, and then vacuum-dried at about 40°C so as to obtain 11.75 g of azetidin-1-yl{8-[(2,6-dimethylbenzyl)amino]-2,3-dimethylimidazo[1,2-a]pyridin-6-yl}methanone citrate.

### Examples 3(2) to 3(4): Preparation of compound I citrate - Crystalline Form C1

Azetidin-1-yl{8-[(2,6-dimethylbenzyl)amino]-2,3-dimethylimidazo[1,2-a]pyridin-6-yl}methanone citrates according to Examples 3(2) to 3(4) of the present invention were obtained, respectively, in substantially the same process as the preparation process of compound I citrate described in Example 3(1) except that the solvent shown in table 3 below was used as a mixed solvent together with acetone as a solvent added together with a solvate to a reactor.

**[Table 3]**

| **Classification** | **solvent** | **Amount of use** |
|---|---|---|
| Example 3(2) | Ethanol | 80 g |
| Example 3(3) | Methanol | 70 g |
| Example 3(4) | IPA | 80 g |

### Example 3(5): Preparation of compound I citrate - Crystalline Form C1

To a reactor, 167.2 g of acetone or 32.1 g of dichloromethane and 10 g of the solvate of compound I obtained in above Preparation Example 2 were added, and then stirred at 10°C to 30°C for 10 minutes. Then, a solution in which 33.4 g of acetone was dissolved in 4.9 g of citric acid was added thereto within 10 minutes, and stirred at the same temperature for one hour. Subsequently, the resulting mixture was cooled down to 0°C to 5°C and further stirred for one hour. The resulting solid was filtered and washed with 20.1 g of acetone, and then vacuum-dried at about 40°C so as to obtain 11.74 g of azetidin-1-yl{8-[(2,6-dimethylbenzyl)amino]-2,3-dimethylimidazo[1,2-a]pyridin-6-yl}methanone citrate.

### Analysis 3 - Results of XRD, DSC, and TGA measurement of Crystalline Form C1

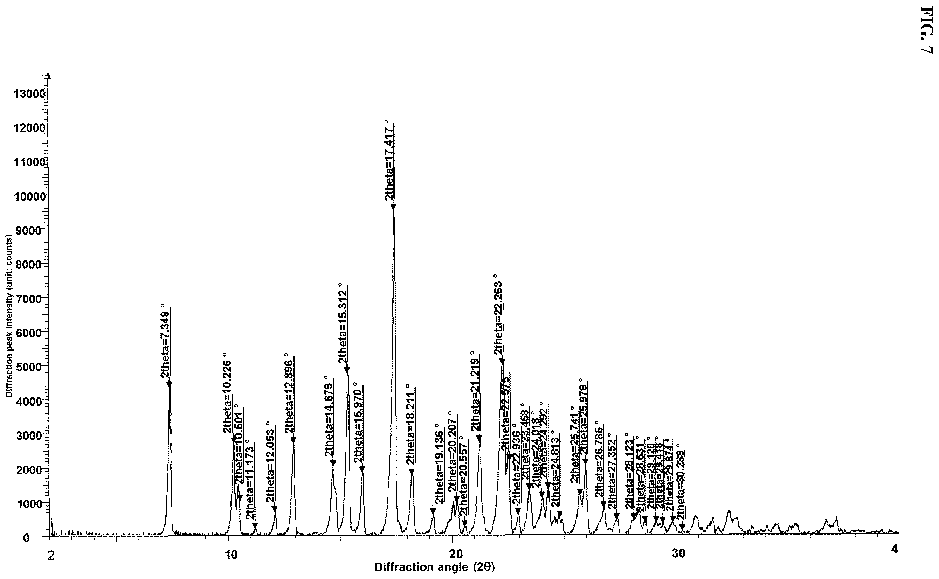

Referring to FIG. 7 and table 4 below, a 2θ value (unit: °) of the XRD pattern of Crystalline Form C1 of the present invention can be confirmed. Specifically, the XRD pattern of Crystalline Form C1 according to the present invention includes a diffraction peak where a 2θ value is 7.35°, 10.23°, 12.90°, 14.68°, 15.31°, 15.97°, 17.42°, 18.21°, 21.22°, 22.26° and 26.00°.

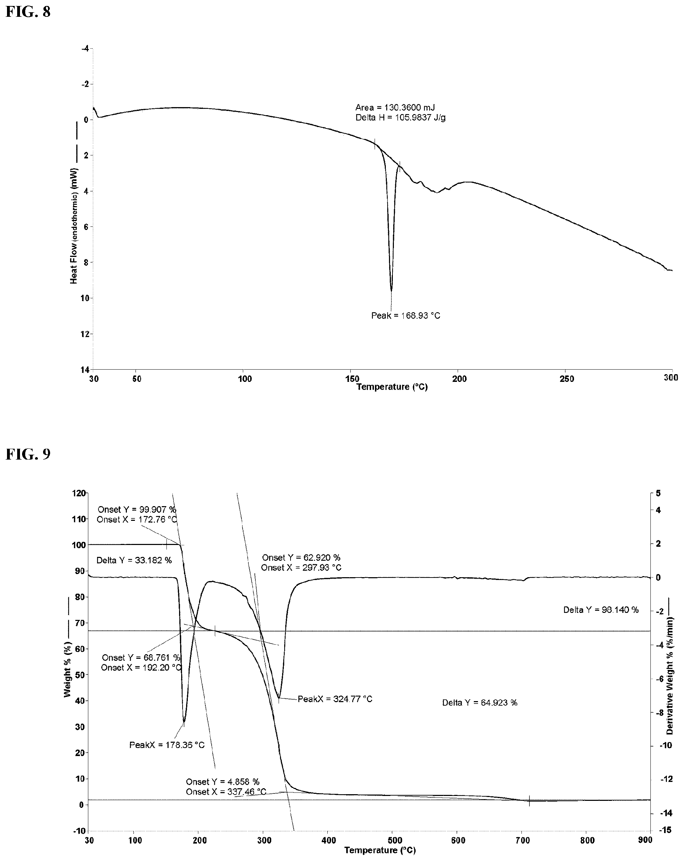

Referring to FIG. 8, Crystalline Form C1 shows a DSC endothermic peak at 168.93°C.

Referring to FIG. 9, as a result of TGA analysis, it is confirmed that Crystalline Form C1 is decomposed in two steps. Specifically, it was found that decomposition occurs in a first step of about 172.76°C to 192.20°C and a second step of about 297.93°C to 337.46°C, and a maximum inflection point to be decomposed is confirmed at 178.36°C and 324.77°C, respectively.

Meanwhile, it can be confirmed through a moisture content that Crystalline Form C1 is anhydrous.

In addition, it was confirmed that Crystalline Form C1 according to the present invention is one in which azetidin-1-yl{8-[(2,6-dimethylbenzyl)amino]-2,3-dimethylimidazo[1,2-a]pyridin-6-yl}methanone and citric acid are bonded at a molar ratio of about 1:1.

**[Table 4]**

| **Diffraction angle (2θ, unit: °)** | **Relative intensity** |
|---|---|
| 7.35 | Strong |
| 10.23 | Moderate |
| 10.50 | Weak |
| 12.05 | Weak |
| 12.90 | Moderate |
| 14.68 | Moderate |
| 15.31 | Strong |
| 15.97 | Moderate |
| 17.42 | Strong |
| 18.21 | Moderate |
| 21.22 | Moderate |
| 22.26 | Strong |
| 23.46 | Weak |
| 24.30 | Weak |
| 25.74 | Weak |
| 26.00 | Moderate |

### Example 4(1): Preparation of compound I citrate - Crystalline Form C2

To a reactor, 167.2 g of acetone, 10 g of the solvate of compound I obtained in above Preparation Example 2 were added, and then stirred at 20°C to 25°C for 10 minutes. Then, a solution in which 33.4 g of acetone was dissolved in 4.9 g of citric acid was slowly added dropwise for 60 minutes or more, and stirred at the same temperature for one hour. Subsequently, the resulting mixture was heated to 50°C to 55°C and further stirred for one hour. The resulting mixture was cooled down to 20°C to 25°C and further stirred for one hour, after which the resulting solid was filtered and washed with 20.1 g of acetone, and then vacuum-dried at 40°C so as to obtain 11.76 g of azetidin-1-yl{8-[(2,6-dimethylbenzyl)amino]-2,3-dimethylimidazo[1,2-a]pyridin-6-yl}methanone citrate.

### Examples 4(2) to 4(4): Preparation of compound I citrate - Crystalline Form C2

Azetidin-1-yl{8-[(2,6-dimethylbenzyl)amino]-2,3-dimethylimidazo[1,2-a]pyridin-6-yl}methanone citrates according to Examples 4(2) to 4(4) of the present invention were obtained, respectively, in substantially the same process as the preparation process of citrate described in Example 4(1) except that the solvent shown in table 5 below was used as a mixed solvent together with acetone as a solvent added together with a solvate to a reactor.

**[Table 5]**

| **Classification** | **Solvent** | **Amount of use** |
|---|---|---|
| Example 4(2) | Ethanol | 70 g |
| Example 4(3) | Methanol | 80 g |
| Example 4(4) | IPA | 80 g |

### Analysis 4 - Results of XRD, DSC, and TGA measurement of Crystalline Form C2

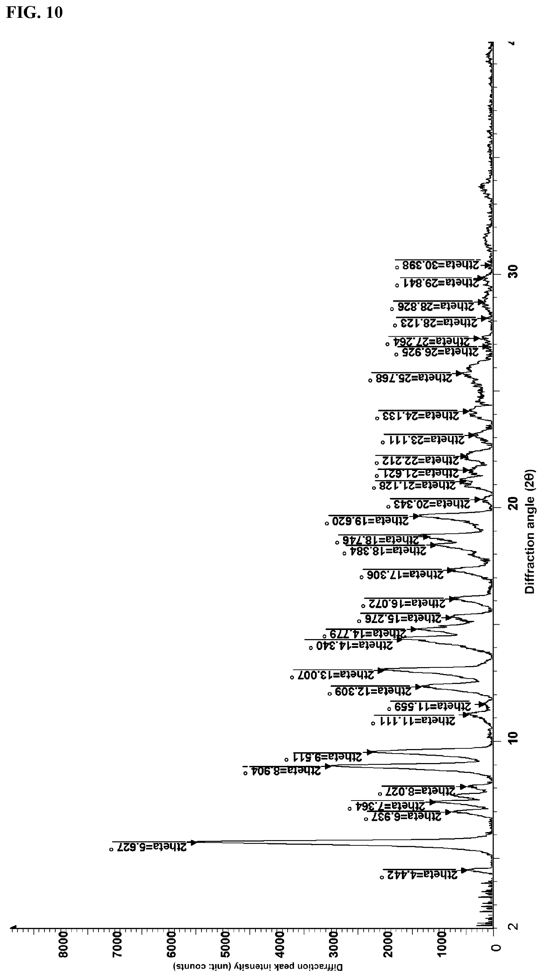

Referring to FIG. 10 and table 6 below, a 2θ value (unit: °) of the XRD pattern of Crystalline Form C2 of the present invention can be confirmed. Specifically, the XRD pattern of Crystalline Form C2 according to the present invention includes a diffraction peak where a 2θ value is 5.63°, 8.90°, 9.51°, 12.31°, 13.01°, 14.34°, 14.80°, 18.38°, 18.75° and 19.62°.

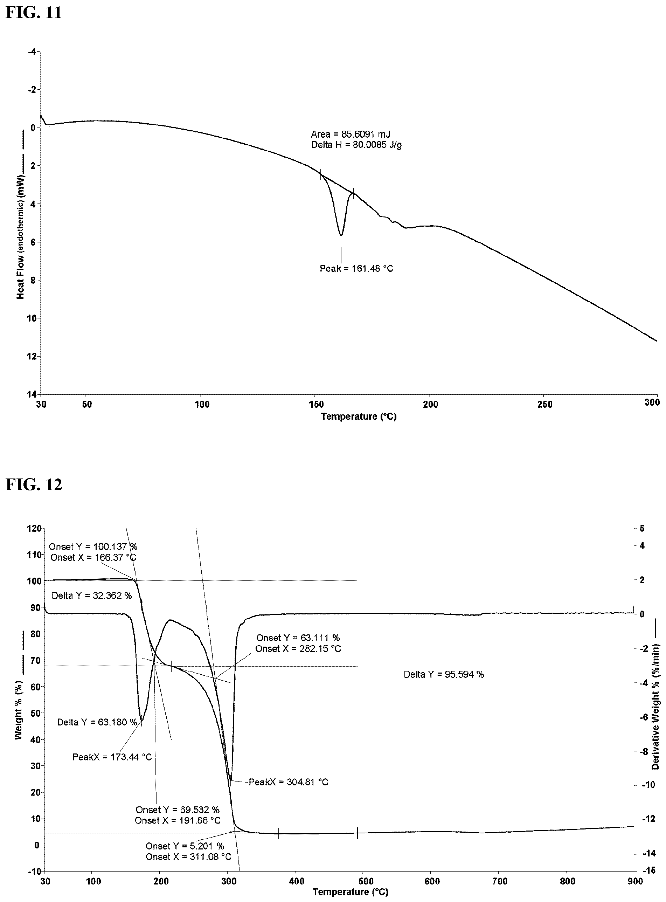

Referring to FIG. 11, Crystalline Form C2 shows a DSC endothermic peak at 161.48°C.

Referring to FIG. 12, as a result of TGA analysis, it is confirmed that Crystalline Form C2 is decomposed in two steps. Specifically, it was found that decomposition occurs in a first step of about 166.37°C to 191.88°C and a second step of about 282.15°C to 311.08°C, and a maximum inflection point to be decomposed is confirmed at 173.44°C and 304.81°C, respectively.

In addition, it was confirmed that Crystalline Form C2 according to the present invention is one in which azetidin-1-yl{8-[(2,6-dimethylbenzyl)amino]-2,3-dimethylimidazo[1,2-a]pyridin-6-yl}methanone and citric acid are bonded at a molar ratio of about 1:1.

**[Table 6]**

| **Diffraction angle (2θ, unit: °)** | **Relative intensity** |
|---|---|
| 4.44 | Weak |
| 5.63 | Strong |
| 6.94 | Weak |
| 7.36 | Weak |
| 8.03 | Weak |
| 8.90 | Strong |
| 9.51 | Strong |
| 12.31 | Moderate |
| 13.01 | Strong |
| 14.34 | Moderate |
| 14.80 | Moderate |
| 16.07 | Weak |
| 17.31 | Weak |
| 18.38 | Moderate |
| 18.75 | Moderate |
| 19.62 | Moderate |
| 21.62 | Weak |
| 22.21 | Weak |
| 23.11 | Weak |

### Example 5(1): Preparation of compound I citrate - Amorphous form

In a reactor, 237.6 g of methanol was added to 10 g of azetidin-1-yl{8-[(2,6-dimethylbenzyl)amino]-2,3-dimethylimidazo[1,2-a]pyridin-6-yl}methanone citrate prepared according to Example 3(1), and then stirred at 20°C to 25°C for 20 minutes to be dissolved. Then, a solid obtained by concentrating was vacuum-dried at about 40°C so as to obtain 9.8 g of amorphous form of azetidin-1-yl{8-[(2,6-dimethylbenzyl)amino]-2,3-dimethylimidazo[1,2-a]pyridin-6-yl}methanone citrate.

### Examples 5(2) and 5(3): Preparation of compound I citrate - Amorphous form

Amorphous forms of azetidin-1-yl{8-[(2,6-dimethylbenzyl)amino]-2,3-dimethylimidazo[1,2-a]pyridin-6-yl}methanone citrates according to Examples 5(2) and 5(3) of the present invention were obtained, respectively, in substantially the same process as the preparation process of compound I citrate described in Example 5(1) except that the alcohol solvent shown in table 7 below was used instead of methanol.

**[Table 7]**

| **Classification** | **Solvent** | **Amount of use** |
|---|---|---|
| Example 5(2) | Ethanol | 710.1 g |
| Example 5(3) | IPA | 3144 g |

### Analysis 5 - Results of DSC and TGA measurement of amorphous form

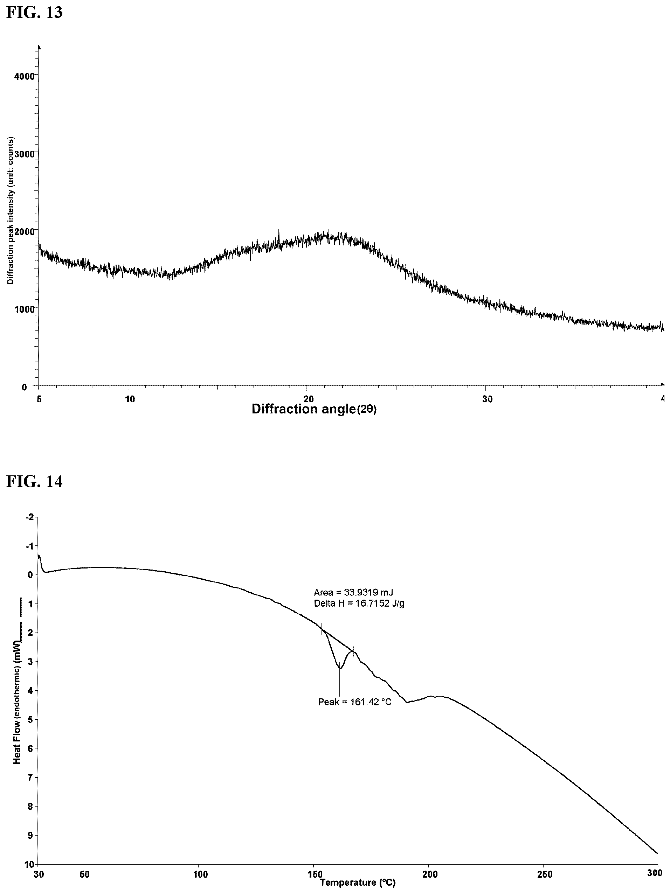

Referring to FIG. 13, it can be confirmed that azetidin-1-yl{8-[(2,6-dimethylbenzyl)amino]-2,3-dimethylimidazo[1,2-a]pyridin-6-yl}methanone citrates according to Examples 5(1) to 5(3) of the present invention are amorphous.

Referring to FIG. 14, the amorphous form of compound I of the present invention shows a DSC endothermic peak at 161.42°C.

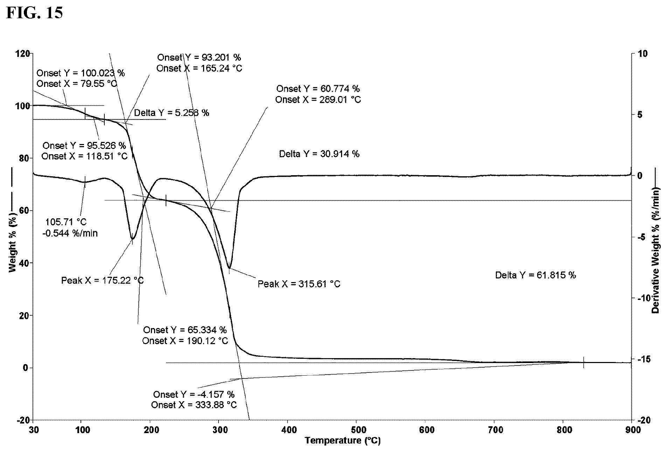

Referring to FIG. 15, as a result of TGA analysis, it is confirmed that the amorphous form of the present invention is decomposed in three steps. Specifically, it was found that decomposition occurs in a first step of about 79.55°C to 118.51°C, a second step of about 165.24°C to 190.12°C, and finally a third step of about 289.01°C to 333.88°C, and a maximum inflection point to be decomposed is confirmed at 105.71°C, 175.22°C, and 315.61°C, respectively.

Meanwhile, it can be confirmed through a moisture content that the amorphous form is also anhydrous.

### Experimental Example 1: Bioavailability evaluation

Bioavailability was evaluated with regard to azetidin-1-yl{8-[(2,6-dimethylbenzyl)amino]-2,3-dimethylimidazo[1,2-a]pyridin-6-yl}methanone citrate according to Example 4(1) of the present invention and azetidin-1-yl{8-[(2,6-dimethylbenzyl)amino]-2,3-dimethylimidazo[1,2-a]pyridin-6-yl}methanone obtained according to Preparation Example 1 as an comparative example. Specific pharmacokinetic experiments and evaluation methods are as follows.

### <Administration and blood collection>

Fasted male beagle dogs were orally administered with capsules at a dose of 10 mg/kg, and bled at 0.08, 0.25, 0.5, 1, 3, 5, 8, and 24 hours after administration. Blood collected in an amount of 3 ml each from the jugular vein was centrifuged to separate plasma, and stored frozen in a cryogenic freezer before analysis.

### <Plasma pretreatment>

Plasma pretreatment was performed by a protein precipitation method. 300 ul of acetonitrile containing an internal standard (200 ng/ml of carbamazepine) was added to 100 ul of a plasma sample and mixed. After sufficiently mixing, the resulting mixture was centrifuged at 12,000 rpm for 10 minutes, after which the supernatant was transferred to an assay vial and injected into a LC-MS/MS to analyze a blood concentration of the compound.

### <LC-MS/MS analysis conditions>

| | | |
|---|---|---|
| | HPLC system | Agilent 1260 series equipped with on-line degasser, binary pump, auto-sampler and column compartment |
| | Column | Waters Xterra MS C18 (2.1x50mm, 3.5um) |
| | Column Oven | 30°C |
| LC | Mobile phase | Linear gradient from 0.1% formic acid/2% |
| | | acetonitrile/98% DI water to 0.1% formic acid/98% |
| | | acetonitrile/2% DI water |
| | Flow rate | 0.35 ml/min |
| | Inj. Volume | 5 ul |
| | MS system | API4000 LC/MS/MS system |
| | Ion source | Turbo V Ion Spray (600°C) |
| MS | Polarity | Positive |
| | MRM | m/z 363>119 |
| | | Internal standard m/z 237> 194 |

### <Pharmacokinetic evaluation>

Parameters of time to maximum plasma concentration (Tₘₐₓ), maximum plasma concentration (Cₘₐₓ), and area under the plasma-concentration time curve (AUC₀₋ₜ, AUC_{0-inf}) were obtained using PK Solution. The results thereof are shown in Table 8 below.

**[Table 8]**

| Item | Unit | **Comparative example: Compound I (free base)** | **Example: Citrate of Compound I** |
|---|---|---|---|
| Tₘₐₓ | (hr) | 5.0 | **1.0** |
| Cₘₐₓ | (ng/ml) | 262.0 | **3033.3** |
| AUC₀₋ₜ | (hr.ng/ml) | 2628.1 | **14968.1** |
| AUC_{0-inf} | (hr.ng/ml) | 2818.0 | **15203.4** |

Referring to above table 8, it can be confirmed that azetidin-1-yl{8-[(2,6-dimethylbenzyl)amino]-2,3-dimethylimidazo[1,2-a]pyridin-6-yl}methanone citrate according to the present invention reached the maximum plasma concentration within a remarkably shorter time than compound I (free base), which was Comparative Example, and the Cmax was remarkably increased 11 times or more than that of Comparative Example, and it can be confirmed that the AUC was also remarkably improved five times or more than that of Comparative Example. In other words, azetidin-1-yl{8-[(2,6-dimethylbenzyl)amino]-2,3-dimethylimidazo[1,2-a]pyridin-6-yl}methanone citrate according to the present invention exhibits a significantly excellent bioavailability that remarkably exceeds a conventionally predictable level.

Accordingly, azetidin-1-yl{8-[(2,6-dimethylbenzyl)amino]-2,3-dimethylimidazo[1,2-a]pyridin-6-yl}methanone citrate according to the present invention may exhibit a very excellent bioavailability that present inventors could not predict, and thus may be used as an active ingredient of the pharmaceutical composition, so as to exhibit a very excellent effect in preventing or treating gastrointestinal inflammatory diseases or gastric acid-related diseases.

### Experimental Example 2: photostability evaluation

With respect to azetidin-1-yl{8-[(2,6-dimethylbenzyl)amino]-2,3-dimethylimidazo[1,2-a]pyridin-6-yl}methanone citrate according to Example 4(1) of the present invention, a sample was thinly spread in a petri dish and then irradiated with Visible having a light amount of 35k lux to reach a total irradiation amount of 1200 k lux under the condition of 25°C and humidity of 60% using a photostability chamber (CARON 6542-2), whereas the sample was thinly spread in the petri dish and then irradiated with UV having a light amount of 35 W to reach a total irradiation amount of 200 watt under the condition of 25°C and humidity of 60% using a photostability chamber (CARON 6542-2), and then discoloration was observed with naked eyes. The results thereof are shown in Table 9 below. It is determined to be suitable only when the white to yellowish off-white powder form is maintained as a solid property.

**[Table 9]**

| Condition | color |
|---|---|
| | **Citrate of Compound I** |
| **Initial value** | White |
| **UV 200 watt** | Off-white |
| **Visible 1200k lux** | Slightly off-white |

Referring to table 9, it can be confirmed that azetidin-1-yl{8-[(2,6-dimethylbenzyl)amino]-2,3-dimethylimidazo[1,2-a]pyridin-6-yl}methanone citrate according to the present invention is maintained to be suitable for a property standard to a degree of off-white color or slightly off-white color even when ultraviolet and visible light are irradiated. As such, it can be seen that azetidin-1-yl{8-[(2,6-dimethylbenzyl)amino]-2,3-dimethylimidazo[1,2-a]pyridin-6-yl}methanone citrate according to the present invention has excellent photostability.

### Experimental Example 3: Evaluation of short-term storage stability (heat/moisture)

Purity of azetidin-1-yl{8-[(2,6-dimethylbenzyl)amino]-2,3-dimethylimidazo[1,2-a]pyridin-6-yl}methanone citrate according to Example 4(1) of the present invention was measured, and then the purity was measured after being left at 60°C for four days. Purity measurements were performed by HPLC. In addition, with regard to azetidin-1-yl{8-[(2,6-dimethylbenzyl)amino]-2,3-dimethylimidazo[1,2-a]pyridin-6-yl}methanone citrate according to Example 4(1) of the present invention, the purity was measured after being left for two days under the condition of 75 to 90% humidity (RH). The results thereof are shown in Table 10 below.

**[Table 10]**

| Condition | Purity (%) |
|---|---|
| | **Citrate of Compound I** |
| Initial value | 99.955 |
| Temperature 60°C, Day 4 | 99.974 |
| Humidity 75~90 %, Day 2 | 99.976 |

Referring to table 10, it can be confirmed that the purity of azetidin-1-yl{8-[(2,6-dimethylbenzyl)amino]-2,3-dimethylimidazo[1,2-a]pyridin-6-yl}methanone citrate according to the present invention is not substantially changed even when being left under the condition of high temperature or left under the condition of high humidity. Thus, it can be seen that azetidin-1-yl{8-[(2,6-dimethylbenzyl)amino]-2,3-dimethylimidazo[1,2-a]pyridin-6-yl}methanone citrate according to the present invention has stability for heat and moisture, respectively.

### Experimental Example 4: Evaluation of long-term storage stability

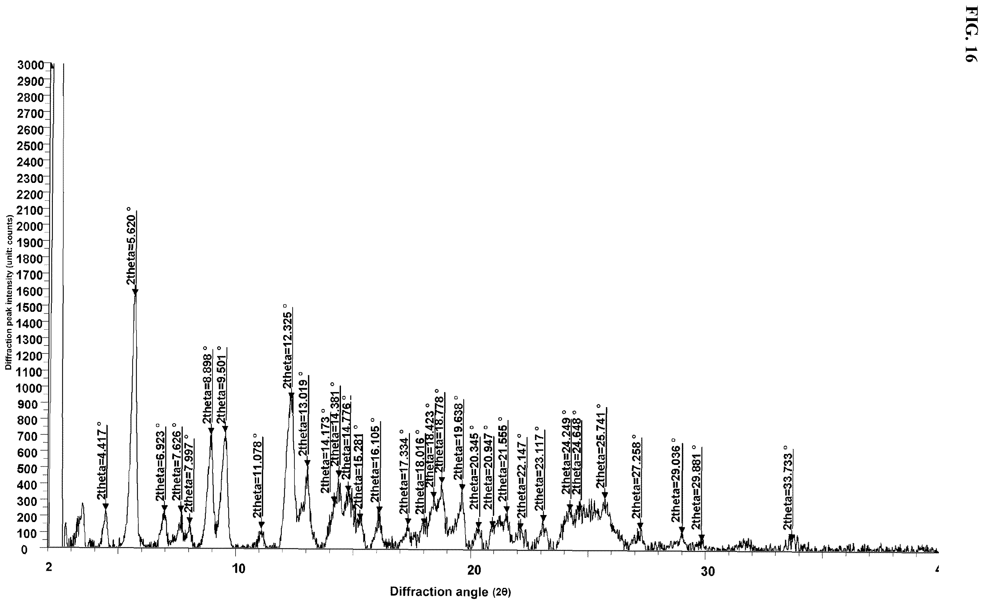

Immediately after preparing azetidin-1-yl{8-[(2,6-dimethylbenzyl)amino]-2,3-dimethylimidazo[1,2-a]pyridin-6-yl}methanone citrate according to Example 4(1) of the present invention, properties were confirmed with naked eyes, and the moisture content, purity, and XRD were measured, respectively. Subsequently, the same was primarily sealed in a polyethylene bag and then secondarily sealed in an aluminum bag, and then the properties were confirmed with naked eyes and the moisture content, purity, and XRD were also measured at 12, 24 and 36 months after being stored under the condition of 25±2°C and 60±5% RH. The results thereof are shown in table 11 below and FIG. 16.

**[Table 11]**

| Test item | Criteria | Storage period | | | |
|---|---|---|---|---|---|
| | | **Immediately after preparation (Initial value)** | **12 months** | **24 months** | **36 months** |
| Property | White to yellowish off-white powder | Suitable | Suitable | Suitable | Suitable |
| Moisture | 1.0% or less | 0.74% | 0.52% | 0.45% | 0.60% |
| Purity | 99.0% or more | 99.96% | 99.96% | 99.97% | 99.96% |
| XRD | (Comparison with initial value) | Same | Same | Same | Same |

Referring to table 11, it can be confirmed that azetidin-1-yl{8-[(2,6-dimethylbenzyl)amino]-2,3-dimethylimidazo[1,2-a]pyridin-6-yl}methanone citrate according to the present invention is stably maintained even at a time point of 36 months after preparation.

In addition, referring to FIG. 16, which is an XRD analysis result after 36 months have elapsed, it can be confirmed that the XRD pattern was maintained without a substantial change, specifically, without a shift of each diffraction peak from that of the citrate of the present invention immediate after preparation as shown in FIG. 10. In other words, it can be confirmed that citrate according to the present invention has very excellent long-term storage stability.

### Experimental Example 5: Evaluation of thermal and moisture stability (accelerated conditions)

Immediately after preparing azetidin-1-yl{8-[(2,6-dimethylbenzyl)amino]-2,3-dimethylimidazo[1,2-a]pyridin-6-yl}methanone citrate according to Example 4(1) of the present invention, properties were confirmed with naked eyes, and the moisture content, purity, and XRD were measured, respectively. Subsequently, the same was primarily sealed in a polyethylene bag and then secondarily sealed in an aluminum bag, and then the properties were confirmed with naked eyes and the moisture content, purity, and XRD were also measured at one and six months after being stored under an accelerated condition of 40±2°C and 75±5% RH. The results thereof are shown in Table 12 below.

**[Table 12]**

| **Test item** | **Criteria** | **Storage period** | | |
|---|---|---|---|---|
| | | **Immediately after preparation (Initial value)** | **1 month** | **6 months** |
| Property | White to yellowish off-white powder | Suitable | Suitable | Suitable |
| Moisture | 1.0% or less | 0.74% | 0.75% | 0.15% |
| Purity | 99.0% or more | 99.96% | 99.97% | 99.96% |
| XRD | (Comparison with initial value) | Same | Same | Same |

Referring to table 12, it can be confirmed that azetidin-1-yl{8-[(2,6-dimethylbenzyl)amino]-2,3-dimethylimidazo[1,2-a]pyridin-6-yl}methanone citrate according to the present invention is stably maintained under the accelerated condition even at a time point of six months after preparation.

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. Azetidin-1-yl{8-[(2,6-dimethylbenzyl)amino]-2,3-dimethylimidazo[1,2-a]pyridin-6-yl}methanone citrate.

2. The azetidin-1-yl{8-[(2,6-dimethylbenzyl)amino]-2,3-dimethylimidazo[1,2-a]pyridin-6-yl}methanone citrate of claim 1, wherein a molar ratio of azetidin-1-yl{8-[(2,6-dimethylbenzyl)amino]-2,3-dimethylimidazo[1,2-a]pyridin-6-yl}methanone and citric acid is 1:0.3 to 1:1.3.

3. The azetidin-1-yl{8-[(2,6-dimethylbenzyl)amino]-2,3-dimethylimidazo[1,2-a]pyridin-6-yl }methanone citrate of claim 1, wherein the citrate is anhydrous.

4. The azetidin-1-yl{8-[(2,6-dimethylbenzyl)amino]-2,3-dimethylimidazo[1,2-a]pyridin-6-yl}methanone citrate of claim 1, wherein the citrate is crystalline.

5. The azetidin-1-yl{8-[(2,6-dimethylbenzyl)amino]-2,3-dimethylimidazo[1,2-a]pyridin-6-yl}methanone citrate of claim 1, wherein the citrate is anhydrous crystalline.

6. The azetidin-1-yl{8-[(2,6-dimethylbenzyl)amino]-2,3-dimethylimidazo[1,2-a]pyridin-6-yl}methanone citrate of claim 1, wherein the azetidin-1-yl{8-[(2,6-dimethylbenzyl)amino]-2,3-dimethylimidazo[1,2-a]pyridin-6-yl}methanone citrate is Crystalline Form A including at least three diffraction peaks where a 2θ (±0.2°) value of an powder X-ray diffraction pattern is selected from the group consisting of 5.46°, 7.14°, 10.61°, 11.82°, 18.27° and 25.77°.

7. The azetidin-1-yl{8-[(2,6-dimethylbenzyl)amino]-2,3-dimethylimidazo[1,2-a]pyridin-6-yl}methanone citrate of claim 6, wherein the azetidin-1-yl{8-[(2,6-dimethylbenzyl)amino]-2,3-dimethylimidazo[1,2-a]pyridin-6-yl}methanone citrate is Crystalline Form A further including at least one diffraction peak where the 2θ (±0.2°) value of the powder X-ray diffraction pattern is selected from the group consisting of 10.93°, 13.11°, 14.15°, 15.84°, 16.35°, 19.79° and 24.21°.

8. The azetidin-1-yl{8-[(2,6-dimethylbenzyl)amino]-2,3-dimethylimidazo[1,2-a]pyridin-6-yl}methanone citrate of claim 6, wherein the azetidin-1-yl{8-[(2,6-dimethylbenzyl)amino]-2,3-dimethylimidazo[1,2-a]pyridin-6-yl}methanone citrate has a differential scanning calorific (DSC) endothermic peak at 88.69°C, 135.61°C and 154.84°C (±0.5°C) when a heating rate is 10°C/min.

9. The azetidin-1-yl{8-[(2,6-dimethylbenzyl)amino]-2,3-dimethylimidazo[1,2-a]pyridin-6-yl}methanone citrate of claim 1, wherein the azetidin-1-yl{8-[(2,6-dimethylbenzyl)amino]-2,3-dimethylimidazo[1,2-a]pyridin-6-yl}methanone citrate is Crystalline Form B including at least three diffraction peaks where a 2θ (±0.2°) value of a powder X-ray diffraction pattern is selected from the group consisting of 7.03°, 7.69°, 9.47°, 13.21°, 14.86° and 21.13°.

10. The azetidin-1-yl{8-[(2,6-dimethylbenzyl)amino]-2,3-dimethylimidazo[1,2-a]pyridin-6-yl}methanone citrate of claim 9, wherein the azetidin-1-yl{8-[(2,6-dimethylbenzyl)amino]-2,3-dimethylimidazo[1,2-a]pyridin-6-yl}methanone citrate has a differential scanning calorific (DSC) endothermic peak at 144.57°C (±0.5°C) when a heating rate is 10°C/min.

11. The azetidin-1-yl{8-[(2,6-dimethylbenzyl)amino]-2,3-dimethylimidazo[1,2-a]pyridin-6-yl}methanone citrate of claim 1, wherein the azetidin-1-yl{8-[(2,6-dimethylbenzyl)amino]-2,3-dimethylimidazo[1,2-a]pyridin-6-yl}methanone citrate is Crystalline Form C1 including at least three diffraction peaks where a 2θ (±0.2°) value of a powder X-ray diffraction pattern is selected from the group consisting of 7.35°, 15.31°, 17.42° and 22.26°.

12. The azetidin-1-yl{8-[(2,6-dimethylbenzyl)amina]-2,3-dimethylimidazo[1,2-a]pyridin-6-yl}methanone citrate of claim 11, wherein the azetidin-1-yl{8-[(2,6-dimethylbenzyl)amino]-2,3-dimethylimidazo[1,2-a]pyridin-6-yl}methanone citrate is Crystalline Form C1 further including at least one diffraction peak where the 2θ (±0.2°) value of the powder X-ray diffraction pattern is selected from the group consisting of 10.23°, 12.90°, 14.68°, 15.97°, 18.21°, 21.22° and 26.00°.

13. The azetidin-1-yl{8-[(2,6-dimethylbenzyl)amino]-2,3-dimethylimidazo[1,2-a]pyridin-6-yl}methanone citrate of claim 11, wherein the azetidin-1-yl{8-[(2,6-dimethylbenzyl)amino]-2,3-dimethylimidazo[1,2-a]pyridin-6-yl}methanone citrate has a differential scanning calorific (DSC) endothermic peak at 168.93°C (±0.5°C) when a heating rate is 10°C/min.

14. The azetidin-1-yl{8-[(2,6-dimethylbenzyl)amino]-2,3-dimethylimidazo[1,2-a]pyridin-6-yl}methanone citrate of claim 1, wherein the azetidin-1-yl{8-[(2,6-dimethylbenzyl)amino]-2,3-dimethylimidazo[1,2-a]pyridin-6-yl}methanone citrate is Crystalline Form C2 including at least three diffraction peaks where a 2θ (±0.2°) value of a powder X-ray diffraction pattern is selected from the group consisting of 5.63°, 8.90°, 9.51° and 13.01°.

15. The azetidin-1-yl{8-[(2,6-dimethylbenzyl)amino]-2,3-dimethylimidazo[1,2-a]pyridin-6-yl}methanone citrate of claim 14, wherein the azetidin-1-yl{8-[(2,6-dimethylbenzyl)amina]-2,3-dimethylimidazo[1,2-a]pyridin-6-yl}methanone citrate is Crystalline Form C2 further including at least one diffraction peak where the 2θ (±0.2°) value of the powder X-ray diffraction pattern is selected from the group consisting of 12.31°, 14.34°, 14.80°, 18.38°, 18.75° and 19.62°.

16. The azetidin-1-yl{8-[(2,6-dimethylbenzyl)amino]-2,3-dimethylimidazo[1,2-a]pyridin-6-yl}methanone citrate of claim 14, wherein the azetidin-1-yl{8-[(2,6-dimethylbenzyl)amino]-2,3-dimethylimidazo[1,2-a]pyridin-6-yl}methanone citrate has a differential scanning calorific (DSC) endothermic peak at 161.48°C (±0.5°C) when a heating rate is 10°C/min.

17. The azetidin-1-yl{8-[(2,6-dimethylbenzyl)amino]-2,3-dimethylimidazo[1,2-a]pyridin-6-yl }methanone citrate of claim 1, wherein the citrate is amorphous.

18. A method for preparing azetidin-1-yl{8-[(2,6-dimethylbenzyl)amino]-2,3-dimethylimidazo[1,2-a]pyridin-6-yl}methanone citrate, the method comprising:
reacting azetidin-1-yl{8-[(2,6-dimethylbenzyl)amino]-2,3-dimethylimidazo[1,2-a]pyridin-6-yl }methanone and citric acid in a presence of at least one solvent selected from an alcohol having one to three carbon atoms, acetone, acetonitrile, tetrahydrofuran (THF), dichloromethane, dimethylformamide (DMF), N-methylpyrrolidone (NMP), and purified water.

19. The method of claim 18, wherein azetidin-1-yl{8-[(2,6-dimethylbenzyl)amino]-2,3-dimethylimidazo[1,2-a]pyridin-6-yl}methanone for a reaction with citric acid in the step is a solvate.

20. The method of claim 18, wherein in the step, citrate where a molar ratio of azetidin-1-yl{8-[(2,6-dimethylbenzyl)amino]-2,3-dimethylimidazo[1,2-a]pyridin-6-yl}methanone and citric acid is 1:0.3 to 1:1.3 is prepared.

21. The method of claim 18, wherein the step includes mixing a first solution including azetidin-1-yl{8-[(2,6-dimethylbenzyl)amino]-2,3-dimethylimidazo[1,2-a]pyridin-6-yl}methanone and a first solvent and a second solution including citric acid and a second solvent,
in which the first solvent is a single solvent of acetone or a mixed solvent including acetone and at least one selected from an alcohol having one to three carbon atoms, acetonitrile, tetrahydrofuran (THF), dichloromethane, dimethylformamide (DMF), and N-methylpyrrolidone (NMP), and
the second solvent is acetone.
